# EUROPÄISCHE PATENTSCHRIFT

(11) **EP 2 366 419 B1**
(45) Veröffentlichungstag und Bekanntmachung des Hinweises auf die Patenterteilung: **16.05.2012**
(21) Anmeldenummer: 10156665.1
(22) Anmeldetag: 16.03.2010
(51) Int. Cl.: A61M 5/44, A61F 7/00

(54) **Beutelwärmer**
Bag warmer
Dispositif de chauffage de récipient

(43) Veröffentlichungstag der Anmeldung: 21.09.2011
(73) Patentinhaber: WWT Technischer Gerätebau GmbH, 70190 Stuttgart (DE)
(72) Erfinder: Theilacker-Beck, Wolfgang, 70176 Stuttgart (DE); Theilacker, Matthias, 70563 Stuttgart (DE); Schmider, Klaus H., 70180 Stuttgart (DE)
(74) Vertreter: Kohler Schmid Möbus

(56) Entgegenhaltungen:
- EP-A1- 2 147 690
- DE-A1-102004 026 446
- US-A1- 2002 147 481
- US-A1- 2004 210 283

## Beschreibung

Die Erfindung betrifft einen Beutelwärmer insbesondere zum Erwärmen von gekühltem Blut auf eine für eine Transfusion geeignete Temperatur, Ein derartiger Beutelwärmer ist geeignet Flüssfgkeifswärmebeutel aufzunehmen, wobei diese Flüssigkeitswärmebeutel ein aus mindestens zwei an bevorzugt vier Begrenzungskanten miteinander verbundenen flexiblen Kunststofffolien gebildetes Durchflussolumen mit einer flachen, im Wesentlichen trapezförmigen Grundform aufweisen, das durch die Begrenzungskanten begrenzt wird. Am Durchlussvolumen sind eine Einlasseitungsöffnung und eine Auslassleitungsöffnung für eine zu wärmende Flüssigkeit vorgesehen.

Ein derartiger Beutelwärmer wird zur Erwärmung von natürlichem Blut für Transfusionszwecke oder bei der Dialyse, aber auch für Transfusionen von Blutplasma oder anderen Flüssigkeiten verwendet.

Blut wird üblicherweise bei einer Temperatur von ca. 4°C aufbewahrt. Vor einer Verwerdung muss dieses Blut daher auf Körpertemperatur von ca. 37°C aufgewärmt werden. Für Transfusionszwecke verwendetes Blut darf keine zu niedrige Temperatur und keine zu höhe Temperatur haben. Insbesondere in Notfällen muss das Erwärmen des Blutes sehr rasch durchgeführt werden, da kein erwärmtes Blut für eventuell auftretende Notfälle bereitgehalten werden kann. In Notfällen muss jedoch nicht nur das Erwärmen des Blutes rasch ermöglicht werden, auch die Handhabung des Blutwärmesystems muss einfach und rasch möglich sein. Die angegebenen Temperaturbedingungen müssen dabei unabhängig von der Durchflussgeschwindigkeit des Blutes durch das Blutwärmesystem eingehalten werden. Diese Durchflussgeschwihdigkeit kann bei. Druckinfusionen über 5 Liter pro Stunde betragen. Zur Vermeidung einer Überhitzung kann daher die Temperatur des Beutelwärmers nur geringfügig über der Körpertemperatur liegen, was zur Folge hat, dass, um eine ausreichende Wärmeübertragun zu erreichen, eine große Wärmeaustauschoberfläche vom Beutelwärmer zur Verfügung gestellt werden muss.

Beutelwärmer sind im Stand der Technik in verschiedenen Ausführungsformen bekannt. Unterschieden werden können Beutelwärmer zum Einlegen eines Flüssigkeitswärmebeutels, die eine aufklappbare, während des Aufwärmens eines eingelegten Flüssigkeitswärmebeutels geschlossene, Kammer aufweisen, in die der Flüssigkeitswärmebeutel eingelegt wird, und Beutelwärmer zum Einführen eines Flüssigkeitswärmebeutels in einen Spalt, der zwischeh zwei mittels Befestigungsmitteln parallel zueinander fixierten Wärmetauscherplatten ausgebildet ist. Die Beutelwärmer mit einer aufklappbaren Kammer sind relativ umständlich zu handhaben und eignen sich daher nur bedingt für den Einsatz in Notfallsituationen.

Beim Erwärmen z.B. von Blut mittels eines Blutwärmesystems kann ein Ausgasen von im Blut gelösten Gasen und damit eine Gasblasenbildung im Flüssigkeitswärmebeutel auftreten. Diese Gasblasen dürfen nicht in den Körper des Patienten, dem das Blut zugeführt wird, gelangen, da sie eine Embolie auslösen könnten. Es sind daher Vorkehrungen zu treffen, um die Gasblasenzuführung zu vermeiden. Letzteres kann z.B. durch visuelle Beobachtung der Blutzuführung geschehen. Aus der US 6,572,641 ist ein gattungsgemäßer Flüssigkeitswärmebeutel bekannt, der zusätzlich zur Einlassleitungsöffnung und der Austassleitungsörfnung eine in ein Gasaufnahmevolumen, das Teil des Durchflussvolumens ist, mündende Entgasungsöffnung aufweist. Diese Entgasungsöffnung ist im Bereich einer beim Flüssigkeitswärmen oben anzuordnenden oberen Begrenzungskante angeordnet und liegt oberhalb der Auslassleitungsöffnung. Dadurch können entstehende Gasblasen, die nach oben steigen, im Gasaufnahmevolumen aufgefangen und durch die Entgasungsöffnung abgeleitet werden. Bei diesem Flüssigkeitswärmebeutel reicht die Entgasungsöffnung relativ weit in das Gasaufnahmevolumen hinein, so dass der Flüssigkeitsspiegel der zu erwärmenden Flüssigkeit oberhalb der Entgasungsöffnung liegt. Letzteres kann zu einem Flüssigkeitsverlust führen, weshalb ein freies Ende eines an die Entgasungsöffnung angeschlossenen Schlauches gegen einen Flüssigkeitsdurchlass abdichtende Flüssigikeitsdurchflussverhinderungsmittel aufweist. Bei diesem Flüssigkeitswärmebeutel sind die Einlassieitungsöffnung, die Auslassleitungsöffnung und die Entgasungsöffnung jeweils an unterschiedlichen Begrenzungskanten, d.h. Seiten des Flüssigkeitswärmebeutels, angeordnet. Dadurch wird die Handhabung des Flüsslgkeitswärmebeutels erschwert, da an die Öffnungen angeschlossene Schläuche ungeordnet aus dem zum Wärmen verwendeten Beutelwärmer herausführen.

Ein Blutwärmesystem mit einem besser handhabbaren gattungsgemäßen Flüssigkeitswärmebeutel ist in der DE 10 2004 026 446 A1 beschrieben. Bei diesem Flüssigkeitswärmebeutel sind die Einlassleitungsöffnung und die Auslassleitungsöffnung an einer beim Flüssigkeitswärmen oben anzuordnenden oberen Begrenzungskante angeordnet. Die obere Begrenzungskante verläuft derart schräg zu ihren angrenzenden Begrenzungskanten, dass zwischen der oberen Begrenzungskante und einer daran angrenzenden Begrenzungskante ein spitzer Winkel eingeschlossen ist. Dieser spitze Winkel schließt einen oberen Durchflussvolumenbereich, ein in den die Auslassleitungsöffnung mündet. Weiter ist eine Flüssigkeitsfluhssführungsnaht vorgesehen, die von einer Verbindung der Kunststofffolien im Bereich des Durchflussvolumens gebildet wird und die obere Begrenzungskante in einen unteren Einlassleitungsöffnungsbereich und in einen oberen Auslassleitungsöffnungsbereich aufteilt. An die Öffnungen dieses Flüssigkeitswärmebeutels ragen geordnet nach oben aus einem verwendeten Beutelwärmer heraus. Jedoch weist dieser Flüssigkeitswärmebeutel keine Entgasungsöffnung auf.

Der Ereindung liegt die Aufgabe zugrunde, einen Beutelwärmer bereitzustellen, welche die Nachteile des Standes der Technik vermeiden. Insbesondere soll die zu erwärmende Flüssigkeit auf ein Temperaturniveau gebracht werden, das gewährleistet, dass eine gewünschte Körpertemperatur eines Patienten gehalten oder in einem möglichst kurzen Zeitraum erreicht wird.

Diese Aufgabe wird durch die in den Patentansprüchen formulierte Lehre gelöst.

Der erfindungsgemäße Beutelwärmer weist einen Empfänger zur Aufnahme von Signalen auf, die analog und/oder digital im Beutelwärmer verarbeitet die Temperatur der Wärmetauscherplatten steuern und/oder regeln. Dies hat den Vorteil, dass die Temperatur der Wärmetauscherplatten ohne zeitlichen Verlust an die Bedürfnisse der vorzunehmenden Transfusion oder Dialyse angepasst werden kann. Mit der erfindungsgemäßen Steuer- und/oder Regelung können vorgegebene und gewünschte Patientenkörpertemperaturen verbessert eingestellt und zeitlich schneller erreicht werden.

In einer bevorzugten Ausgestaltung der Erfindung geben die vom Beutelwärmer aufgenommenen Signale Körpertemperaturen eines Patienten wieder und die Signale steuern und/oder regeln die Heizleistung der Wärmetauscherplatten derart, dass eine am Beutelwärmer für einen Patienten einstellbare Körpertemperatur über eine Erwärmung der zu applizierenden Flüssigkeit, wie Blut oder Blutplasma, mit einer Temperatur erfolgt die größer, gleich oder kleiner als die eingestellte Körpertemperatur ist. Dies hat den Vorteil, dass eine Unterkühlung eines Patienten bzw. eine unzulässige Erhöhung einer vorgegebenen Patientenkörpertemperatur mit der erfindungsgemäßen Steuer- und/oder Regelung sicher verhindert werden kann. Die gewünschten Patientenkörpertemperaturen werden schnellstmöglich ohne Zeitverzögerung angeglichen. Dies fördert das Wohlbefinden eines Patienten während einer Transfusion oder Dialyse.

Es versteht sich, dass die Temperaturen zur Erwärmung der zu applizierenden Flüssigkeit über den Zeitraum der Transfusion oder Dialyse mit der erfindungsgemäßen Steuer- und/oder Regelung ständig verändert werden könnten. Je nach Bedarf wird die Heizleistung der Wärmetauscherplatten erhöht oder erniedrigt. Ein Temperaturbereich zwischen T = 30° Celsius und 45° Celsius kann an den Oberflächen der Wärmetauscherplatten eingestellt werden. Die in den Flüssigkeitswärmebeuteln strömende Flüssigkeit kann dadurch schnellstmöglich erwärmt bzw. gekühlt werden. Die einem Patienten über eine Infusionsleitung zugeführte Flüssigkeit kann über vom Beutelwärmer vorgegebene Zeitbereiche Temperaturen zwischen T = 30° Celsius und T = 45° Celsius annehmen und dies auch dann, wenn die am Beutelwärmer voreingestellte Körpertemperatur zwischen diesen Temperaturwerten liegt. Entscheidend für die Flüssigkeitstemperatur ist die an den Beuteilwärmer vom Patienten, der die gewünschte Transfusion oder Dialyse erhält, übermittelte Körpertemperatur, die an die am Beutelwärmer voreingestellte Patientenkörpertemperatur schnellstmöglich angeglichen werden soll.

Am Beutelwärmer selbst ist ein Display oder eine Eingabeeinrichtung für die Einstellung einer gewünschten Patentenkörpertemperatur vorgesehen. Diese vorgegebene Pätientenkörpertemperatur wird in ein elektrischen beziehungsweise elektronisches Signal umgewandelt und im Beutelwärmer derart verarbeitet, dass es mit von einem Patienten empfangenen Signal beziehungsweise Signalen verglichen beziehungsweise verarbeitet werden kann, derart dass die Flüssigkeitstemperaturen aus dem Beutelwärmer bestmöglich an die gewünschte am Beutelwärmer voreingestellte Patientenkörpertemperatur angeglichen werden kann. Es versteht sich, dass die am Beutelwärmer einzustellende Patientenkörpertemperatur auch alternativ oder ausschließlich über eine drahtlose oder drahtgebundene Fernsteuerung erfolgen kann.

Die Temperatur der zu applizierenden Flüssigkeit zu einem Patienten wird erfindungsgemäß von zwei unabhängig voneinander arbeitenden Auswerteeinheiten erfasst und die Signale, die vom Beutelwärmer über den Empfänger vom Patienten erfasst werden und Körpertemperaturwerte darstellen, werden mit dem vorgegebenen am Beutelwärmer eingestellten Körpertemperaturwert verglichen und aus dem Vergleich wird eine Heizleistung für die Wärmetauscherplatten generiert, damit die zu applizierende Flüssigkeit mit einer Temperatur dem Patienten zugeführt werden kann, die gewährleistet, dass er während einer Transfusion oder Dialyse nicht unterkühlt, oder die Patlentenkörpertemperatur unzulässigerweise stark angehoben wird. Erfindungsgemäß wird dadurch die jeweils am Patienten gemessene Körpertemperatur schnellstmöglich an die am Beutelwärmer eingestellte Körpertemperatur angepasst und dies über einen völlautomatisierten Regel- und/oder Steuerkreis zwischen dem Patienten und dem Beutelwärmer.

Über Ventile an der Einlass- und Auslassleitung am Beutelwärmer oder am Flüssigkeitswärmebeutel kann die Flüssigkeitstemperatur bzw. die Flüssigkeitszufuhr und/oder die Entgasung der zu applizierenden Flüssigkeit weiter gesteuert und geregelt werden. An der Auslassleitung können auf deren weiteren Vertauf zum Patienten verschiedene bei Infusionen oder Transfusionen übliche Vorrichtungen an der Auslassleitung vorgesehen sein, z.B. eine Schlauchheizung oder eine Tropfkammer.

Die Erfindung wird nachfolgend anhand von Ausführungsbeispielen unter Bezugnahme auf die Zeichnungen näher erläutert.

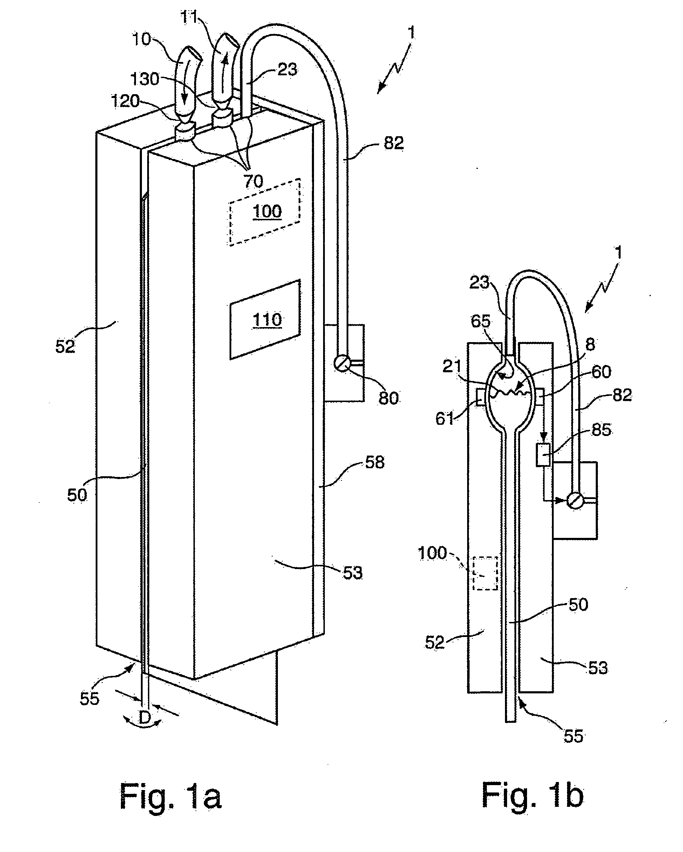

Die Figuren 1a und 1b zeigen einen erfindungsgemäßen Beutelwärmer mit einem eingeführten erfindungsgemäßen Flüssigkeitswärmebeutel, wobei Figur 1b einen schematischen Querschnitt durch den Beutelwärmer mit einem eingeführten Flüssigkeitswärmebeutel zeigt.

Die Darstellungen der Zeichnungen zeigen den erfindungsgemäßen Gegenstand stark schematisiert und sind nicht maßstäblich zu Verstehen. Die einzelnen Bestandteile des erfindungsgemäßen Gegenstandes sind so dargestellt, dass ihr Aufbau gut, gezeigt werden kann.

In den Figuren 1a und 1b ist ein erfindungsgemäßer Beutelwärmer 1 mit einem eingeführten erfindungsgemäßen Flüssigkeitswärmebeutel 50 dargestellt, wobei Figur 1b einen schematischen Querschnitt durch den Beutelwärmer mit einem eingeführten Flüssigkeitswärmebeutel 50 zeigt. Zum Aufwärmen der im Flüssigkeitswärmebeutel 50 aufzuwärmenden Flüssigkeit weist der Beutelwärmer 1 zwei im Wesentlichen rechteckige Wärmetauscherplatten 52,53 als Heizelemente auf. Die Wärmetauscherplatten 52,53 bilden im Inneren des Beutelwärmers 1 jeweils eine im Wesentlichen flächige Wärmeaustauschoberfläche aus. Die Wärmetauscherplatten 52,53 sind mit ihren Wärmeaustauschoberflächen derart beabstandet sich gegenüberliegend angeordnet, dass zwischen den Wärmeaustauschoberflächen ein Spalt 55 mit einer Spaltbreite D von einigen Millimetern zur Aufnahme des Flüssigkeitswärmebeutels 50 ausgebildet ist.

Die Wärmetauscherplatten 52,53 sind an einer ersten Längsseitenkante der Wärmetauscherplatten 52,53 mit Befestigungsmitteln 58 derart miteinander befestigt, dass die Wärmeaustauschoberflächen durch die Befestigungsmittel 58 im Wesentlichen parallel zueinander fixiert sind. Dabei ist der Spalt 55 entlang einer zweiten Längsseitenkante und den Schmalseitenkanten der Wärmetauscherplatten offen, wodurch das Einlegen des Flüssigketswärmebeutels 50 zwischen die Wärmetauscherplatten 52,53 einfach möglich ist. Die Befestigungsmittel 58 sind derart federnd ausgebildet, dass eine in dem in den Beutelwärmer 1 eingeführten Flüssigkeitswärmbeutel 50 auftretende Druckveränderung zu einer Veränderung der Spaltbreite D führt. Dabei führt eine Druckerhöhung zu einer Aufweitung und eine Druckverminderung zu einer Einengung des Spaltes 55, was durch einen gebogenen Doppelpfeil in der Figur 1a symbolisch dargestellt ist.

Im Bereich der Wärmeaustauschoberflächen ist ein Flüssigkeitsstandsensor 60 zur Detektion eines Flüssigkeitsstandes 21 in dem in den Beutelwärmer eingeführten Flüssigkeitswarmebeutel 50 vorgesehen. Der Flüssigkeitsstandsensor 60 kann z.B. als ein Lichtdetektor in der Wärmeaustauschoberfläche einer der Wärmetäuscherplatten 52,53 ausgebildet sein, wobei im auf dem Füssigkeitsstandsensor 60 gegenüberliegenden Bereich der Wärmeaustauschoberfläche der anderen Wärmetauscherplatte 52 eine Lichtquelle 61 z.B. eine Leuchtdiode positioniert sein kann. Von der Lichtquelle 61 emittiertes Licht wird dabei je nach Flüssigkeitsstand mehr oder weniger gedämpft vom Flüssigkeitsstandsensor 60 detektiert. Im an die obere Schmalseitenkante der Wärmetauscherplatte 52,53 angrenzenden Bereich der Wärmetauscherplatten 52,53 ist eine Kavität 65 zur Formgebung des Gasaufnahmevolumens 8 des Flüssigkeitswärmebeutels 50 ausgebildet. Der Flüssigkeitswarmebeutel 50 ist also so zwischen den Wärmetauscherplatten 52,53 derart positioniert, dass das Gasaufnahmevolumen 8 des Flüssigkeitswärmebeutels 50 im Bereich der Kavität 65 angeordnet ist. Auch der Flüssigkeitsstandsensor 60 ist im Bereich dieser Kavität 65 positioniert, so dass der Flüssigkeitsstand 21 im Flüssigkeitswärmebeutel 50 im Bereich des Gasaufnahmevolumens 8 detektiert werden kann.

Der Flüssigkeitssensor 60 kann auch eine andere Ausgestaltung haben und ist beispielsweise ergänz- oder ersetzbar durch einen Ultraschallsensor oder durch eine andere Gasblasenerkennungstechnik.

Der Beutelwärmer 1 weist an den oberen Schmalseitenkante der Wärmetauscherplatten 52,53 drei Nutansätze 70 auf, die zu der Einlassleitungsöffnung 10 und zu der Auslassleitungsöffnung 11 und zu der Entgasungsöffnung 23 des in den Beutelwärmer 1, eingeführten Flüssigkeitswärmebeutels 50 derart komplementär ausgeformt sind, dass eine an den Flüssigkeitswärmebeutel 50 angeschlossene Einlaufleitung und eine an den Flüssigkeitswärmebeutel 50 angeschlossene Auslassleitung und eine an den Flüssigkeitswärmebeutel 50 angeschlossene Entgasungsleitung, während der Flüssigkeitswärmebeutel 50 in den Beutetwärmer eingeführt ist, in den Nutansätzen 70 formschlüssig gehalten sind. Am Beutetwärmer 1 ist ein Entgasungsventil 80 zum Anschluss der Entgasungsöffnung 23 des in den Beutelwärmer 1 eingeführten Flüssigkeitswärmebeutels 50 über eine Schlauchleitung 82 vorgesehen. Mittels des Entgasungsventils 80 kann also die Entgasungsöffnung 23 variabel geöffnet oder verschlossen werden. Der Flüssigkeitsstandsensor 60 und das Entgasungsventil 80 sind an eine Steuereinheit 85 angeschlossen. Die Steuereinheit 85 ist eingerichtet, bei einer durch den Flüssigkeitsstandsensor 60 detektierten Überschreitung eines vorbestimmten Flüssigkeitsstandes 21 im Gasaufnahmevolumen 8 des Flüssigkeitswarmebeutels 50 das Entgasungsventil 80 zum Verschluss der Entgasungsöffnung 23 zu steuern und bei einer durch den Flüssigkeitsstandsensor 60 detektierten Unterschreitung des vorbestimmten Flüssigkeitsstandes 21 im Gasaufnahmevolumen 8 des Flüssigkeitswärmebeutels 50 das Entgasungsventil 80 zum Öffnen der Entgasungsöffnung 23 zu steuern. Der dabei auftretende Signalfluss ist in Figur 1b durch die Pfeilspitzan der Verbindunge zwischen Flüssigkeitsstandsensor 60 und Steüereinheit 85 sowie zwischen Steuereinheit 85 und Entgasungsventil 80 symbolisiert.

Der Beutelwärmer 1 weist zusätzlich einen im Beutelwärmer 1 integrierten Empfänger 100 zur Aufnahme von Signalen auf, die im Beutelwärmer 1 analog und/oder digital weiterverarbeitet werden. Über die empfangenen und aufbereiteten Signale kann die Temperatur der Wärmetauscherplatten 52, 53 gesteuert und/oder geregelt werden. Die Signale geben beispelsweise Körpertemperaturen wieder, die beispielsweise über ein Fieberthermometer (drahtlos oder drahtgebunden) an den Beutelwärmer 1 übermittelt werden. Am Beutelwärmer 1. selbst ist ein Display 110 zur Einstellung einer Patientenkörpertemperatur vorgesehen, die über die durchzuführende Transfusion möglichst nur geringfügig verändert werden soll. Im Idealfall soll die Pätiehtenkörpertemperatur im gesamten Zeitraum der applizierten Transfusion konstant gehalten werden. Dazu werden die übertragenen Signale vom Patienten zum Beutelwärmer 1 mit dem elektronisch bereitgestellten Signal am Beutelwärmer 1, das der vorgegebenen Patientenkörpertemperatur entspricht, verglichen und derart aufgearbeitet, dass mit dem aufgearbeiteten Signal die Beheizung der Wärmetauscherplatten 52, 53 gesteuert und geregelt wird. Die Steuerung und Regelung sieht vor, dass die zu applizierende Flüssigkeit erwärmt, gekühlt oder in der erfassten Temperatur konstant gehalten werden kann.

An der Einlassleitung 10 und an der Auslassleitung 11 sind Ventile 120, 130 vorgesehen, über die unabhängig voneinander die Zufuhr der zu applizierenden Flüssigkeit in und/oder aus dem Beutelwarmer 1. unterbrochen werden kann.

Dies ist eine zusätzliche Maßnahme wie die Temperatur einer zu applizierenden Flüssigkeit beeinflusst werden kann. Zusätzlich lassen sich Blasenbildungen in der Flügssigkeitszufuhr zum Patienten wirksam verhindern.

Ein Beutelwärmer 1 zur Erwärmung von gekühlten Flüssigkeiten auf eine für eine Transfusion geeignete Temperatur im medizinischen Bereich weist eine Öffnung zum Einführen eines Flüssigkeitswärmebeutels 50 auf, der im Beutelwärmer 1 über Heizelemente, die im Wesentlichen über zwei rechteckige Wärmetauscherplatten 52, 53 ausgebildet sind, aufgeheizt werden kann. Über die Wärmetauscherplatte 52, 53 ist auch eine Kühlung des Flüsslgkeitswärmebeutels 50 möglich. In dem Beutelwärmer 1 ist mindestens ein Empfänger 100 zur Aufnahme von drahtlos übertragenen Signalen und/oder über Leitungen übertragenen Signalen vorgesehen, die im Beutelwärmer 1 von Bauelementen zu weiterverarbeitbaren digitalen und/oder analogen Signalen weiterverarbeitet werden, wobei diese analogen und/oder digitalen Werte geeignet sind, die Temperatur der Wärmetauscherplatten 52, 53 zu steuern und/oder zu regeln. Über Ventile 120, 130, die an einer Einlass- und/oder einer Auslassleitung 10, 11 am Beutelwärmer 1 oder am Flüssigkeitswärmebeutel 50 vorgesehen sind, wird die einem Patienten zu applizierende Flüssigkeit in und/oder aus dem Beutelwärmer 1 freigegeben oder abgesperrt.

Die Erfindung beschränkt sich nicht auf die vorstehend angegebenen Ausführungsbeispiele.

## Patentansprüche

1. Beutelwärmer zum Einführen eines Flüssigkeitswärmebeutels (50) mit Heizelementen, die zwei im wesentlichen rechteckige Wärmetauscherplatten (52, 53) mit jeweils einer im Wesentlichen flächigen Wärmeaustauschoberfläche ausbilden, wobei die Wärmetauscherplatten (52, 53) mit deren Wärmeaustauschoberfläche derart beabstandet sich gegenüberliegend angeordnet sind, dass zwischen den Wärmeaustauschoberflächen ein Spalt (55) mit einer Spaltbreite (D) zur Aufnahme eines Flüssigkeitswärmebeutels (50) ausgebildet ist, **dadurch gekennzeichnet, dass** der Beutelwärmer (1) einen Empfänger (100) zur Aufnahme von Signalen aufweist, die analog und/oder digital im Beutelwärmer (1) weiterverarbeit bar sind, nm die Temperatur der Wärmetauscherplatten (52, 53) zu steuern und/oder zu regeln.

2. Beutelwärmer nach Anspruch 1, **dadurch gekennzeichnet, dass** die vom Beutelwärmer (1) aufgenommenen Signale Körpertemperaturen eines Patienten wiedergeben, und dass die Heizleistung der Wärmetauscherplatten (52, 53) durch die Signale derart steuer- und/oder regelbar ist, dass eine am Beutelwärmer (1) für einen Patienten einstellbare Körpertemperatur über eine Erwärmung der zu applizierenden Flüssigkeit, wie Blut oder Blutplasma, mit einer Temperatur erfolgt, die größer, gleich oder kleiner als die eingestellte Körpertemperatur ist.

3. Beutelwärmer nach Anspruch 2, **dadurch gekennzeichnet, dass** die Temperatur zur Erwärmung der zu applizierenden. Flüssigkeit über den Zeitraum einer Transfusion oder Dialyse ander bar ist.

4. Beütelwärmer nach Anspruch 2 oder 3, **dadurch gekennzeichnet, dass** am Beutelwärmer 1 ein Display (110) vorgesehen ist, über das eine Körpertemperatur für einen Patienten voreinstellbar ist.

5. Beutefwärmer nach einem der Ansprüche 2 bis 4, **dadurch gekennzeichnet, dass** die Temperatur der zu applizierende Flüssigkeit von mindestens zwei voneinander unabhängigen Auswerteeinheiten mit den über die Signale empfangenen Korpertemperaturwerte eines Patienten vergleichbar ist und dass die Auswerteeinheiten die Temperatur der zu applizierende Flüssigkeit derart steuern und/oder regeln, dass sich die über die Signale empfangenen Temperaturwerte vom Patienten an die am Beutelwärmer (1) eingestellte Körpertemperatur angleichen.

6. Beutelwarmer nach einem der Anspruche 1 bis 5, **dadurch gekennzeichnet, dass** der Beutelwärmer (1) Einlass- und Auslassleitungen (10, 11) aufweist, an denen Ventile (120, 130) vorgesehen sind, die die Zufuhr der zu applizierenden Flüssigkeit in und/oder aus dem Beutelwärmer (1) freigeben oder sperren.

## Claims

1. Bag heating means for inserting a liquid heating bag (50) comprising heating elements which form two substantially rectangular heat exchanger plates (52, 53) each having a substantially flat heat exchanger surface, wherein the heat exchanger surfaces of the heat exchanger plates (52, 53) are disposed opposite to each other and are spaced apart from each other such that a gap (55) of a gap width (D) is formed between the heat exchanger surfaces for receiving a liquid heating bag (50), **characterized in that** the bag heating means (1) has a receiver (100) for receiving signals that can be further processed in an analogue and/or digital manner in the bag heating means (1) for controlling and/or regulating the temperature of the heat exchanger plates (52, 53).

2. Bag heating means according to claim 1, **characterized in that** the signals received by the bag heating means (1) reflect body temperatures of a patient, and the heating power of the heat exchanger plates (52, 53) can be controlled and/or regulated by the signals in such a manner that a body temperature that can be set for a patient on the bag heating means (1) is realized through heating of the liquid to be applied, such as blood or blood plasma, with a temperature that is larger, equal or smaller than the set body temperature.

3. Bag heating means according to claim 2, **characterized in that** the temperature for heating the liquid to be applied can be changed over the period of a transfusion or dialysis.

4. Bag heating means according to claim 2 or 3, **characterized in that** a display (110) is provided on the bag heating means 1, by means of which a body temperature can be preselected for a patient.

5. Bag heating means according to any one of the claims 2 through 4, **characterized in that** the temperature of the liquid to be applied can be compared by means of at least two evaluation units, which are independent of each other, with the body temperature values of a patient, which were received via the signals, and the evaluation units control and/or regulate the temperature of the liquid to be applied in such a manner that the temperature values of the patient, which are received via the signals, match the body temperature adjusted on the bag heating means (1).

6. Bag heating means according to any one of the claims 1 through 5, **characterized in that** the bag heating means (1) has inlet and outlet lines (10, 11) comprising valves (120, 130) which enable or block supply of the liquid to be applied into and/or out of the bag heating means (1).

## Revendications

1. Dispositif de réchauffage de récipients, conçu pour l'insertion d'un récipient (50) de réchauffage de fluides et muni d'éléments chauffants qui se présentent comme deux plaques (52, 53) d'échange de chaleur pour l'essentiel rectangulaires, respectivement pourvues d'une surface d'échange thermique essentiellement plane, lesdites plaques (52, 53) d'échange de chaleur étant placées en vis-à-vis mutuel avec espacement, par leur surface d'échange thermique, de manière à réserver un interstice (55) de largeur (D) entre les surfaces d'échange thermique, en vue de recevoir un récipient (50) de réchauffage de fluides, **caractérisé par le fait que** le dispositif (1) de réchauffage de récipients est équipé d'un récepteur (100) dévolu à la réception de signaux pouvant être traités ultérieurement dans ledit dispositif (1) de réchauffage de récipients, en mode analogique et/ou numérique, afin de commander et/ou de réguler la température des plaques (52, 53) d'échange de chaleur.

2. Dispositif de réchauffage de récipients, selon la revendication 1, **caractérisé par le fait que** les signaux reçus par ledit dispositif (1) de réchauffage de récipients reproduisent des températures corporelles d'un patient ; et **par le fait que** la puissance chauffante des plaques (52, 53) d'échange de chaleur peut être commandée et/ou régulée, par l'intermédiaire desdits signaux, de façon telle qu'une température corporelle pouvant être réglée pour un patient, sur ledit dispositif (1) de réchauffage de récipients, découle d'un réchauffage du fluide devant être administré, tel que du sang ou du plasma sanguin, à une température supérieure, égale ou inférieure à la température corporelle réglée.

3. Dispositif de réchauffage de récipients, selon la revendication 2, **caractérisé par le fait que** la température, assignée au réchauffage du fluide à administrer, peut être modifiée pendant la période d'une transfusion ou d'une dialyse.

4. Dispositif de réchauffage de récipients, selon la revendication 2 ou 3, **caractérisé par le fait qu'**un affichage (110), permettant le préréglage d'une température corporelle assignée à un patient, est prévu sur ledit dispositif (1) de réchauffage de récipients.

5. Dispositif de réchauffage de récipients, selon l'une des revendications 2 à 4, **caractérisé par le fait que** la température du fluide à administrer peut être comparée, par au moins deux unités d'interprétation indépendantes l'une de l'autre, aux valeurs de température corporelle d'un patient qui sont reçues au moyen des signaux ; et **par le fait que** lesdites unités d'interprétation commandent et/ou régulent ladite température du fluide à administrer, de façon telle qu'un équilibre s'instaure entre lesdites valeurs de température, reçues par le patient au moyen des signaux, et la température corporelle réglée sur ledit dispositif (1) de réchauffage de récipients.

6. Dispositif de réchauffage de récipients, selon l'une des revendications 1 à 5, **caractérisé par le fait que** ledit dispositif (1) de réchauffage de récipients comporte des conduits (10, 11) d'admission et d'évacuation sur lesquels sont prévues des valves (120, 130) par lesquelles la délivrance du fluide à administrer, audit dispositif (1) de réchauffage de récipients et/ou hors de ce dernier, est autorisée ou interdite.
